(19) Europäisches Patentamt
European Patent Office
Office européen des brevets

(11) Veröffentlichungsnummer: **0 006 535**
**B1**

(12) # EUROPÄISCHE PATENTSCHRIFT

(45) Veröffentlichungstag der Patentschrift: **01.12.82**

(51) Int. Cl.[3]: **C 08 G 59/62,** C 08 G 59/38, C 08 J 9/04, C 08 L 63/00

(21) Anmeldenummer: **79101928.4**

(22) Anmeldetag: **13.06.79**

(54) **Lagerfähige, feste Mischung zur Herstellung hydrolysebeständiger Kunststoffe auf Epoxidharzbasis, ihre Verwendung zur Herstellung solcher Kunststoffe und so erhaltene Kunststoffe.**

(30) Priorität: **22.06.78 CH 6821/78**

(43) Veröffentlichungstag der Anmeldung: **09.01.80 Patentblatt 80/1**

(45) Bekanntmachung des Hinweises auf die Patenterteilung: **01.12.82 Patentblatt 82/48**

(84) Benannte Vertragsstaaten: **CH DE FR GB IT**

(56) Entgegenhaltungen:
**DE-A-1 595 518**
**DE-B-1 645 556**
**GB-A-1 445 994**
**GB-A-1 462 536**
**US-A-3 373 121**
**US-A-4 075 260**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Patentabteilung Postfach, CH-4002 Basel (CH)**

(72) Erfinder: **Lohse, Friedrich, Prof. Dr., Buchenstrasse 23, CH-4104 Oberwil (CH)**
Erfinder: **Gutekunst, Ferdinand, Oberdorfstrasse 13, CH-4125 Riehen (CH)**
Erfinder: **Schmid, Rolf, Dr., Weihermattweg 18, CH-4460 Gelterkinden (CH)**
Erfinder: **Schmitter, André, Dr., Rue des Alpes 7, F-68220 Hegenheim (FR)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Lagerfähige, feste Mischung zur Herstellung hydrolysebeständiger Kunststoffe auf Epoxidharzbasis, ihre Verwendung zur Herstellung solcher Kunststoffe und so erhaltene Kunststoffe

Die Erfindung betrifft eine lagerfähige, feste Mischung, welche zur Herstellung hydrolysebeständiger Kunststoffe auf Epoxidharzbasis dient und mindestens zwei verschiedene Epoxidharze, ein Trisphenol und einen Härtungsbeschleuniger enthält, ferner die Verwendung dieser Mischung zur Herstellung von Kunststoffen und so erhaltene Kunststoffe.

Die Umsetzung von Tris-(glycidyloxyphenyl)-propan mit mehrwertigen Phenolen in Gegenwart eines alkalischen Katalysators ist aus der DAS 1030021 bekannt. Die Mischungen können zusammen mit Füllstoffen unter Druck und Hitze verpresst werden, sie können auch als Imprägnierharze für Textilmaterial verwendet werden. Die Umsetzungsprodukte bilden zähe, harte Harze. Ihre Flexibilität und bzw. oder ihre Glasumwandlungstemperaturen entsprechen jedoch nicht immer den gewünschten Anforderungen. Auch ist die Herstellung der Glycidyloxyharze ziemlich aufwendig, da grosse Mengen organischer Lösungsmittel dazu eingesetzt werden müssen, welche wieder zu entfernen sind.

Aus der US-A-4075260 ist eine Mischung aus bei Raumtemperatur festem, langkettigem Epoxidharz ohne Estergruppen, einem estergruppenfreien, cyclische Strukturen aufweisenden Epoxidharz, einem Härter, wobei als solche Amine, Säureanhydride und insbesondere Dicyandiamid genannt werden, sowie einem Beschleuniger bekannt. Die Mischung ergibt bei der Härtung Produkte, deren Wärmebeständigkeit für viele Zwekke ungenügend ist. In der GB-A-1462536 wird eine härtbare Mischung eines Epoxidharzes mit einem 3 phenolische OH-Gruppen aufweisenden Härter und einem Härtungsbeschleuniger beschrieben. Die Mischung kann zusätzlich noch Bisphenole enthalten.

In der GB-A-1445994 wird eine härtbare Zusammensetzung aus einem oder mehreren Epoxidharzen und einem Poly(hydroxystyrol) beschrieben. Sie kann ausserdem Katalysatoren und andere Zusätze enthalten. Die gehärteten Produkte zeichnen sich durch Sprödigkeit aus.

In Anbetracht der unübersehbaren Literatur betreffend Gemische aus Epoxidharz, Härter und Katalysator kann weder eine einzelne der angeführten Publikationen noch ihre Kombination die erfindungsgemässe Mischung nahelegen. Diese behebt die oben angegebenen Nachteile. Sie ist dadurch gekennzeichnet, dass sie

(a) ein langkettiges, lineares, estergruppenfreies, bei Raumtemperatur festes Epoxidharz mit einem Epoxidäquivalentgewicht $>700$, vorzugsweise $>1000$,

(b) eine estergruppenfreie, cyclische Strukturen und 2 oder 3 Epoxidgruppen im Molekül aufweisende Verbindung mit einem Epoxidäquivalentgewicht von 100 bis 400, vorzugsweise 150 bis 300, wobei die Komponenten (a) und (b) in einem solchen Mengenverhältnis vorhanden sind, dass auf 1 Gesamtepoxidäquivalent 0,05 bis 0,3 Äquivalente von (a) und 0,7 bis 0,95 Äquivalente, vorzugsweise 0,8 bis 0,95 Äquivalente, von (b) stammen,

(c) ein Trisphenol der Formel I

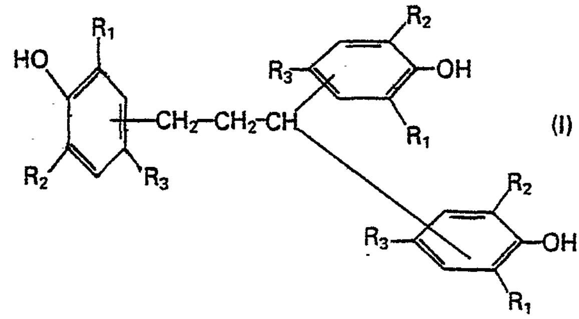

(I)

in welcher $R_1$ und $R_2$ unabhängig voneinander Wasserstoff, Methyl oder Halogen und $R_3$ Wasserstoff oder Methyl bedeuten, in einer Menge, dass auf jedes Epoxidäquivalent 0,5 bis 1,1 Äquivalente der phenolischen Hydroxyverbindung entfallen, wobei bis zu 70 Äquivalent-% davon durch phenolische Bishydroxyverbindungen ersetzt sein können, und

(d) einen Härtungsbeschleuniger

enthält.

Als Komponente (a) kommen vor allem in bekannter Weise hergestellte Umsetzungsprodukte aus n Mol Bisphenol A mit n+1 Mol Epichlorhydrin, wobei n eine Zahl von 5 bis 16, vorzugsweise 7 bis 13 bedeutet, in Betracht. Als Komponente (b) kommen vorzugsweise Diglycidyläther von Bisphenol A und Bisphenol F, Epoxyphenol- oder -kresolnovolake, Triglycidylisocyanurat oder Di- oder Triglycidylderivate von Hydantoinen in Betracht. Beispiele für geeignete Hydantoine sind N,N'-Diglycidylverbindungen von in 5-Stellung durch niedere Alkylgruppen, wie Methyl- oder Äthylgruppen, oder einen Tetra- oder Pentamethylengruppe substituierten Hydantoinen oder an den N-Atomen in 1- oder 3-Stellung glycidylierte zweikernige Hydantoine, deren Ringe über die 3- bzw. 1-Stellung durch eine Methylengruppe oder durch eine 1,3-Propylengruppe, die in 2-Stellung durch eine niedrige Alkylgruppe und/oder eine Glycidyloxygruppe substituiert sein kann, verbunden sind.

Als Komponente (c) werden vorzugsweise solche Verbindungen der Formel I verwendet, worin $R_1$ und $R_2$ Wasserstoff und $R_3$ entweder Wasserstoff oder Methyl bedeuten oder $R_1$ und $R_2$ für Methyl oder Brom und $R_3$ für Wasserstoff stehen.

Die Herstellung dieser Triphenole wird in der DAS 1030021 beschrieben. Bei dieser Herstellungsweise, bei welcher man 3 Mol eines Phenols in Gegenwart eines sauren Katalysators mit einem Mol Acrolein umsetzt, entstehen bei Verwendung von Phenolen mit 2 oder 3 kernaktiven Stellen neben den Triphenolen auch höhere Polyphenole, Pentaphenole und Heptaphenole. Ein Teil dieser Triphenole, nämlich bis zu 70 Äquivalent-%, kann

durch phenolische Bishydroxyverbindungen ersetzt sein. Als solche kommen die bekannten Bisphenole, wie Bisphenol A und F, und auch Gemische solcher Bisphenole mit halogenierten Bisphenolen in Frage.

Als Komponente (d) können zur Beschleunigung der Härtung des Harz/Härter-Gemisches tertiäre Amine, Imidazole, Alkalialkoholate oder quaternäre Ammoniumsalze, wie Tetramethylammoniumchlorid, verwendet werden.

Die erfindungsgemässen Mischungen können auf übliche Weise hergestellt werden, indem man z. B. Harz und Härter bei erhöhter Temperatur, z. B. zwischen 100 und 150°C, zusammenmischt und nach dem Abkühlen und Zerkleinern den Härtungsbeschleuniger beifügt. Falls das Gemisch sofort nach seiner Herstellung verarbeitet werden soll, kann der Beschleuniger auch in der Wärme dem Harz und dem Härter beigemischt werden. Das Gemisch weist in der Wärme ein gutes Fliessverhalten auf.

Die Zusammensetzungen eignen sich bei Zusatz eines Treibmittels auch zur Herstellung von Schäumen.

Zur Herstellung des Schaums werden den Mischungen solche Treibmittel zugesetzt, die bei Raumtemperatur noch fest sind und deren Zersetzungspunkte zwischen 80 und 220°C liegen. Diese können allein oder als Gemische zugesetzt werden. Es können Sulfonylhydrazide, z. B. Benzolsulfonylhydrazid, p-Toluolsulfonylhydrazid oder 4,4'-Oxy-dibenzolsulfonsäurehydrazid, weiterhin Substanzen wie Azodicarbonamide oder α,α'-Azoisobuttersäurenitril verwendet werden. Vorzugsweise dient Azodicarbonamid als Treibmittel. Die Dichte der neuen Schaumstoffe wird weitgehend durch das Gewichtsverhältnis von Reaktionsmischung zu Treibmittel bestimmt; sie kann von 0,1 bis 1,0 variieren. Vorzugsweise werden 0,5–7% Treibmittel verwendet.

Zum Erzielen einer homogenen Porenstruktur kann noch eine oberflächenaktive Substanz in Mengen von 0,1% bis 1%, bezogen auf die Gesamtmenge des Reaktionsgemisches, beigefügt werden. Die Mischung wird dabei gewöhnlich in eine Form gegeben, die nötigenfalls verschlossen werden kann. Die Schaumbildung, Formgebung und Aushärtung erfolgt in einem Ofen, vorzugsweise bei 160–200°C, während etwa ½ bis 6 h. Der gebildete Schaumstoffkörper kann aus der Form entnommen werden, wenn er bei der angewendeten Temperatur formbeständig ist. Man kann ihn nötigenfalls bei der gleichen oder bei höheren Temperaturen nachhärten, um dadurch vorteilhaftere Gebrauchseigenschaften zu erreichen.

Die lagerstabilen und homogenen Mischungen können in Form von Prepregs, Sinterpulvern, Pressmassen und Klebepulvern durch Erhitzen auf Temperaturen über 120°C ohne Lösungsmittel zu Laminaten, Schäumen, Pressartikeln und Verklebungen mit guter Hydrolysebeständigkeit, Wärmeformbeständigkeit, und Flexibilität verarbeitet werden.

Beispiel 1

180 g (1,0 Äq) Epoxidnovolak mit einem Epoxidgehalt von 5,56 Äq/kg, 182 g (0,1 Äq) eines Diglycidyläthers von vorverlängertem Bisphenol A, hergestellt aus etwa 6 Mol Diglycidyläther von Bisphenol A und 5 Mol Bisphenol A in Gegenwart von Tetramethylammoniumchlorid, mit einem Epoxidgehalt von 0,55 Äq/kg werden mit 57 g (0,5 Äq) Bisphenol A und 48 g (0,5 Äq) 1,1,3-Tris-(hydroxyphenyl)-propan, einer Verbindung der Formel I, worin $R_1 = R_2 = R_3 =$ Wasserstoff bedeuten, bei 140°C gemischt. Darauf werden 0,93 g (0,2%) 1-Methylimidazol zugemischt und die Mischung sofort auf ein kaltes Al-Blech ausgegossen. Das erhaltene Produkt ist mahlbar und weist folgende Eigenschaften auf:

| | |
|---|---|
| Glasumwandlungstemperatur (DSC-1, 16°C/min) | = 48°C |
| Gelierzeit bei 160°C | = 180 sec. |

(«DSC-1» ist ein von der Firma Perkin-Elmer Corp. vertriebenes Differential Scanning Calorimeter).

Nach Härtung der Mischung werden folgende Torsionsklebefestigkeiten auf Aluminium gemessen:

| | |
|---|---|
| Härtung während 30 min bei 160°C | = 76 N/mm² |
| Härtung während 2 h bei 140°C und 2 h bei 210°C | = 82 N/mm² |

Herstellung von 1,1,3-Tris-(hydroxyphenyl)-propan:

1410 g (15 Mol) Phenol werden mit 0,9 ml 33 gew.-%iger Salzsäure versetzt und auf 45 bis 55°C erhitzt. Nach Erreichen dieser Temperatur werden 56 g (1 Mol) Acrolein tropfenweise zugefügt, wobei eine schwach exotherme Reaktion eintritt. Die Temperatur wird dabei durch Eiskühlung bei höchstens 65°C gehalten. Nach 10 min ist alles Acrolein zugegeben, worauf 1 h bei 100°C nachgerührt wird. Anschliessend wird das nicht reagierte Phenol an der Wasserstrahlpumpe abdestilliert. Hierzu muss die Temperatur laufend erhöht werden, bis nach etwa 3 h bei 200°C kein Destillat mehr übergeht. Der Rückstand erstarrt bei Raumtemperatur zu einer hellrotbraunen glasigen Masse. Der Erweichungsbereich liegt bei 40 bis 50°C.

Die Auswertung der gelchromatographischen Analyse ergibt, dass das Produkt ein Gemisch von mindestens 3 Komponenten darstellt, wobei Tris-(hydroxyphenyl)-propan 68 Gew.-% ausmacht.

Daneben sind enthalten 200 Gew.-% Pentaphenol und 10 Gew.-% Heptaphenol nebst geringen Anteilen höhermolekularer Produkte.

Den Polyphenolen können folgende Strukturformeln zugeschrieben werden:

Trisphenol:

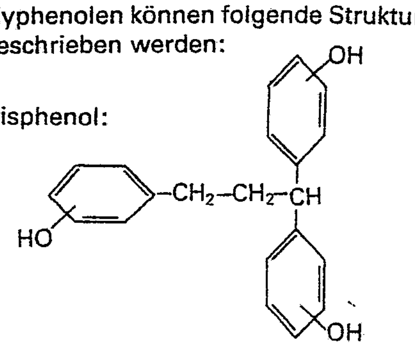

Pentaphenol:

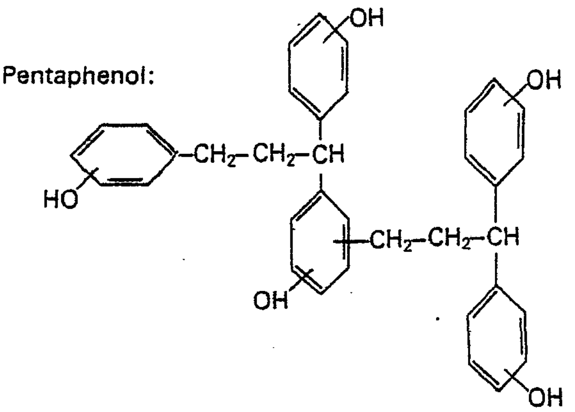

Beispiel 2

182 g (0,1 Äq) eines Diglycidyläthers von vorverlängertem Bisphenol A, hergestellt wie in Beispiel 1 angegeben, mit einem Epoxidgehalt von 0,55 Äq/kg, 167 g (0,9 Äq) Diglycidyläther von Bisphenol A mit einem Epoxidgehalt von 5,4 Äq/kg werden mit 96 g (0,9 Äq) der Trihydroxyverbindung der Formel I, worin $R_1 = R_2 = R_3$ = Wasserstoff bedeuten, bei 140°C gemischt. 0,34 g (0,1%) 1-Methylimidazol werden zugemischt, worauf die Mischung sofort auf ein kaltes Al-Blech ausgegossen wird. Das erhaltene Produkt ist mahlbar und weist folgende Eigenschaften auf:

| | |
|---|---|
| Glasumwandlungstemperatur (DSC-1, 16°C/min) | = 39°C |
| Gelierzeit bei 160°C tel quel | = 460 sec |
| Gelierzeit bei 160°C nach 52 Tagen bei RT | = 300 sec |
| Gelierzeit bei 160°C nach 52 Tagen bei 40°C | = 90 sec |

Nach Härtung während 2 h bei 160°C misst man eine Torsionsklebefestigkeit auf Aluminium von 70 N/mm².

Beispiel 3

180 g (0,1 Äq) eines Diglycidyläthers von vorverlängertem Bisphenol A, hergestellt wie in Beispiel 1 angegeben, mit einem Epoxidgehalt von 0,55 Äq/kg, 55,5 g (0,3 Äq) Diglycidyläther von Bisphenol A mit einem Epoxidgehalt von 5,4 Äq/kg, 108 g (0,6 Äq) Epoxidphenolnovolak mit einem Epoxidgehalt von 5,56 Äq/kg werden mit 22,8 g (0,2 Äq) Bisphenol A, 99,6 g (0,4 Äq) Tetrabrombisphenol A und 28,8 g (0,3 Äq) der Trihydroxyverbindung der Formel I, worin $R_1 = R_2 = R_3$ = Wasserstoff bedeuten, bei 160°C gemischt. Darauf wird 1,0 g (0,2%) 1-Methylimidazol zugemischt und die Mischung sofort nachher auf ein kaltes Al-Blech ausgegossen. Das erhaltene Produkt ist bei Raumtemperatur mahlbar und weist folgende Eigenschaften auf:

| | |
|---|---|
| Glasumwandlungstemperatur (DSC-1, 16°C/min | = 49°C |
| Gelierzeit bei 160°C | = 180 sec. |

Das gehärtete Produkt ist flammhemmend und weist folgende Torsionsklebefestigkeiten auf Aluminium auf:

| | |
|---|---|
| Nach Härtung während 30 min bei 160°C | = 77 N/mm² |
| Nach Härtung während 2 h bei 140°C und 2 h bei 210°C | = 79 N/mm². |

Beispiel 4

Bei Raumtemperatur (20°C) werden 182 Teile eines höhermolekularen Diphenylolpropandiglycidylätherharzes (Epoxidgehalt 0,55 Äq/kg, vgl. Beispiel 1) und 180 Teile eines Epoxidnovolakharzes, welches 5,56 Epoxidäquivalent pro kg besitzt, gemischt. Dem Harzgemisch werden anschliessend fein gemahlen 57 Teile Bisphenol A sowie 48 Teile eines Kondensationsproduktes von Phenol und Acrolein (Formel I, $R_1 = R_2 = R_3$ = H) beigegeben. Das Gemisch wird so lange auf 160°C gewärmt, bis sich die phenolhaltigen Produkte gelöst haben. Anschliessend werden in einem Kneter 0,2% eines oberflächenaktiven Produktes auf der Basis von Polysiloxan, ferner 7 Gew.-% Azodicarbonamid beigemischt. Zuletzt werden dem Gemisch 0,9 Teile Methylimidazol beigegeben. Das so erhaltene Gemisch wird sofort ausgeladen und mit Trockeneis sehr rasch auf Raumtemperatur abgekühlt. 18 g des Produktes werden in eine auf 150°C vorgewärmte Stahlform mit den Abmessungen 64×34×16 mm eingebracht und verschlossen. Nach 1 h Härtung wird ein Schaumkörper der Dichte 0,4 g/cm³ entnommen, welcher eine feine Porenstruktur besitzt. Seine Erweichungstemperatur liegt bei 123°C.

Beispiel 5

In einem Kneter, bei dem die Manteltemperatur auf 70°C eingestellt ist, werden folgende Stoffe gemischt:

55,5 Teile Diphenylolpropandiglycidyläther (Epoxidäquivalent 5,4), 182 Teile eines höhermolekularen Diphenylolpropandiglycidylätherharzes (Epoxidäquivalent 0,55, vgl. Beispiel 1), 108 Teile Epoxynovolak mit einem Epoxidäquivalent von 5,56, 28,8 Teile eines Kondensationsproduktes aus Phenol und Acrolein (Formel I, $R_1 = R_2 = R_3$ = H), 22,8 Teile Bisphenol A, 100 Teile Tetrabrombisphenol A, 1 Teil 1-Methylimidazol, 0,2% eines oberflächenaktiven Mittels auf der Basis von Polysiloxan und 7% Azodicarbonamid.

Die Vorverlängerung im Kneter wird während 30 min fortgesetzt. Anschliessend wird das pastenförmige Produkt aus dem Kneter herausgenommen und gekühlt. In eine auf 160°C vorgewärmte Form aus Stahl mit den Abmessungen 65×34×16 mm werden 18 g des in einer Mühle gemahlenen Produktes eingebracht. Die Form wird verschlossen und während 1 h bei 160°C gehalten. Nach erfolgter Härtung und anschliessender Abkühlung wird ein Schaumkörper der Dichte 0,4 g/cm³ entnommen. Dieser zeigt eine feine und homogene Porenstruktur. Seine Erweichungstemperatur auf einer TMS-Apparatur gemessen liegt bei 120°C. (TMS bedeutet Thermo-Mechanical-Scanning-Apparatur, die von der Firma Perkin-Elmer vertrieben wird.)

Beispiel 6

364 g (0,4 Äq) einer vorverlängerten Bisphenol-A-diglycidylverbindung mit einem Epoxidgehalt von 1,1 Äq/kg, 100 g (0,6 Äq) einer Hydantoinverbindung mit folgender Konstitution

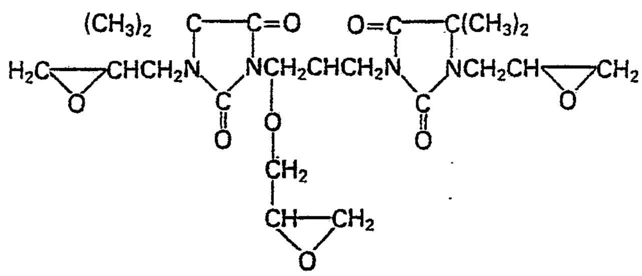

mit einem Epoxidgehalt von 6,0 Äq/kg werden mit 24,8 g (0,4 Äq) 2,6-Dihydroxytoluol und 57,6 g (0,6 Äq) 1,1,3-Tris-(hydroxyphenyl)-propan bei 140°C gemischt. 2,7 g (0,05%) einer 10% wässrigen Lösung von Tetramethylammoniumchlorid werden zugemischt, worauf die Mischung sofort auf ein Al-Blech ausgegossen wird. Das erhaltene Produkt ist mahlbar und weist folgende Eigenschaften auf:

Glasumwandlungstemperatur («DSC-1», 16°C/min = 52°C
Gelierzeit bei 160°C = 10 min

Nach der Härtung der Mischung werden folgende Torsionsklebefestigkeiten auf Aluminium gemessen:

Härtung während 15 min bei 160°C = 29 $N/mm^2$
Härtung während 60 min bei 160°C = 85 $N/mm^2$

Beispiel 7

546 g (0,6 Äq) einer vorverlängerten Bisphenol-A-diglycidylverbindung mit einem Epoxidgehalt von 1,1 Äq/kg, 546 g (3,26 Äq) einer Hydantointriglycidylverbindung entsprechend Beispiel 6, 76,8 g (0,8 Äq) Tris-(hydroxyphenyl)-propan, 24,8 g (0,4 Äq) 2,6-Dihydroxytoluol, 147,4 g (2,68 Äq) 1,3-Dihydroxybenzol, 2,7 g (0,2%) «Aerosil 200»* und 10,0 g (0,1%) Tetramethylammoniumchlorid (10%ige wässrige Lösung) werden zusammengegeben und bei 140°C zusammengeschmolzen. Die erhaltene glasige Masse ist mahlbar und weist folgende Eigenschaften auf:

Glasumwandlungstemperatur («DSC-1», 6°C/min = 44°C
Gelierzeit bei 160°C = 6½ min
Gelierzeit bei 180°C = 3½ min
Torsionsklebefestigkeit auf Al nach Härtung während 1 h bei 160°C = 68 $N/mm^2$
Torsionsklebefestigkeit auf Al nach Härtung während 2 h bei 160°C = 81 $N/mm^2$

* «Aerosil» ist ein der DEGUSSA geschütztes Warenzeichen. Mit «Aerosil 200» wird eine hochdisperse Kieselsäure, hergestellt durch Hydrolyse von Siliciumtetrachlorid in einer Knallgasflamme, bezeichnet. Sie enthält über 99,8% $SiO_2$.

**Patentansprüche**

1. Lagerfähige, feste Mischung zur Herstellung von hydrolysebeständigem Kunststoff auf Epoxidharzbasis, dadurch gekennzeichnet, dass sie

(a) ein langkettiges, lineares, estergruppenfreies, bei Raumtemperatur festes Epoxidharz mit einem Epoxidäquivalentgewicht >700,

(b) eine estergruppenfreie, cyclische Strukturen und 2 oder 3 Epoxidgruppen im Molekül aufweisende Verbindung mit einem Epoxidäquivalentgewicht von 100 bis 400, wobei die Komponenten (a) und (b) in einem solchen Mengenverhältnis vorhanden sind, dass auf 1 Gesamtepoxidäquivalent 0,05 bis 0,3 Äquivalente von (a) und 0,7 bis 0,95 Äquivalente von (b) stammen,

(c) ein Trisphenol der Formel I

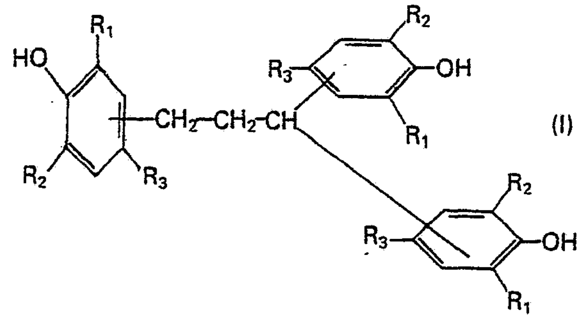
(I)

in welcher $R_1$ und $R_2$ unabhängig voneinander Wasserstoff, Methyl oder Halogen und $R_3$ Wasserstoff oder Methyl bedeuten, in einer Menge, dass auf jedes Epoxidäquivalent 0,5 bis 1,1 Äquivalente der phenolischen Hydroxyverbindung entfallen, wobei bis zu 70 Äquivalent-% davon durch phenolische Bishydroxyverbindungen ersetzt sein können, und

(d) einen Härtungsbeschleuniger

enthält.

2. Mischung gemäss Anspruch 1, dadurch gekennzeichnet, dass sie als Komponente (d) ein tertiäres Amin, ein Imidazol, ein Alkalialkoholat oder ein quaternäres Ammoniumsalz enthält.

3. Mischung gemäss Anspruch 1, dadurch gekennzeichnet, dass sie ausserdem ein Treibmittel enthält.

4. Verwendung der Mischung gemäss Anspruch 1 zur Herstellung eines Kunststoffes, dadurch gekennzeichnet, dass man sie bei einer Temperatur von mehr als 120°C reagieren lässt.

5. Kunststoff auf Epoxidharzbasis, hergestellt aus einer Mischung gemäss Anspruch 1.

**Claims**

1. A storable, solid mixture for the preparation of a plastic which is based on epoxide resin and is stable to hydrolysis, which comprises (a) a long-chain, linear epoxide resin, which is free from ester groups, is solid at room temperature and has an epoxide equivalent weight of >70, (b) a compound which is free from ester groups, contains cyclic structures and 2 or 3 epoxide groups in the molecule and has an epoxide equivalent weight of 100 to 400, the components (a) and (b) being present in a ratio such that, per 1 total epoxide equivalent, 0.05 to 0.3 equivalent originates from (a) and 0.7 to 0.95 equivalent originates from (b),

(c) a trisphenol of the formula I

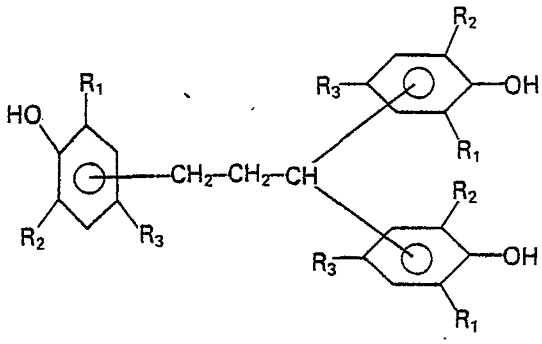

(I)

in which $R_1$ and $R_2$ independently of one another are hydrogen, methyl or halogen and $R_3$ is hydrogen or methyl, in an amount such that, to each epoxide equivalent, there are 0.5 to 1.1 equivalents of the phenolic hydroxyl compound, whereby up to 70 equivalent-% thereof can be replaced by phenolic bishydroxyl compounds, and (d) a curing accelerator.

2. A mixture according to claim 1, which contains, as component (d), a tertiary amine, an imidazole, an alkali metal alcoholate or a quaternary ammonium salt.

3. A mixture according to claim 1, which additionally contains a blowing agent.

4. The use of a mixture according to claim 1 for the preparation of a plastic, wherein the mixture is allowed to react at a temperature of more than 120°C.

5. A plastic which is based on epoxide resin and has been prepared from a mixture according to claim 1.

**Revendications**

1. Mélange solide, se conservant bien, pour la préparation de matières plastiques, stables à l'hydrolyse, à base de résines époxydiques, mélange caractérisé en ce qu'il contient:

a) une résine époxydique à longue chaîne, linéaire, dépourvue de groupes esters, solide à la température ambiante et ayant un poids équivalent d'époxyde supérieur à 700,

b) un composé dépourvu de groupes esters, contenant dans sa molécule des structures cycliques et deux ou trois groupes époxy et ayant un poids équivalent d'époxyde de 100 à 400, les composantes (a) et (b) étant présentes en un rapport tel que, sur 1 équivalent d'époxyde en tout, de 0,05 à 3 équivalent provienne de (a) et de 0,7 à 0,95 équivalent provienne de (b),

c) un tris-phénol répondant à la formule (I)

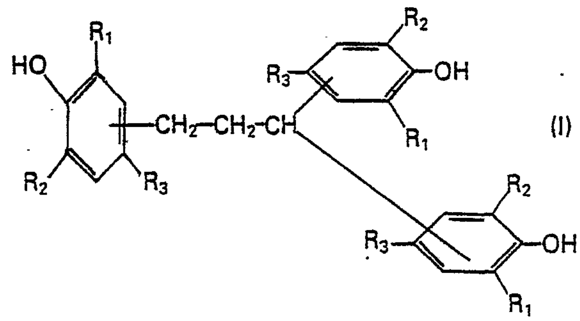

(I)

dans laquelle $R_1$ et $R_2$ représentent chacun, indépendamment l'un de l'autre, l'hydrogène, un méthyle ou un halogène et $R_3$ représente l'hydrogène ou un méthyle, en une quantité telle qu'il y ait de 0,5 à 1,1 équivalent du composé hydroxy phénolique pour chaque équivalent d'époxyde, le composé hydroxy phénolique pouvent être remplacé, en une proportion d'eau plus 70% en équivalents, par des composés bis-hydroxy phénoliques, et

d) un accélérateur de durcissement.

2. Mélange selon la revendication 1, caractérisé en ce qu'il contient, comme composante (d), une amine tertiaire, un imidazole, un alcoolate de métal alcalin ou un sel d'ammonium quaternaire.

3. Mélange selon la revendication 1, caractérisé en ce qu'il contient en outre un porogène.

4. Application du mélange selon la revendication 1 à la préparation d'une matière plastique, application caractérisée en ce qu'on fait réagir ledit mélange à une température de plus de 120°C.

5. Matière plastique à base d'une résine époxydique, qui a été préparée à partir d'une mélange selon la revendication 1.